# EUROPEAN PATENT APPLICATION

(11) **EP 1 736 482 A1**
(43) Date of publication of application: **27.12.2006**
(21) Application number: 05291319.1
(22) Date of filing: 20.06.2005
(51) Int. Cl.: C07K 14/52, C12N 5/00, C12N 15/62

(54) **Recombinant trimeric 4-1BBL**

(71) Applicant: INSERM (Institut National de la Santé et de la Recherche Medicale), 75013 Paris (FR)
(72) Inventor: Lang, François, 44300 Nantes (FR); Rabu, Catherine, 44000 Nantes (FR)
(74) Representative: Vialle-Presles, Marie José

(57) **Abstract**

The invention relates to recombinant 4-1 BBL polypeptides comprising the whole extracellular domain of native 4-1 BBL, and a cross-linkable peptide domain. These recombinant polypeptides are able to associate into homotrimers. Said homotrimers have an inhibitory effect on the proliferation of T-lymphocytes.

These homotrimers can be complexed or linked to a solid support through their cross-linkable domains. These cross-linked recombinant 4-1 BBL homotrimers have a costimulatory activity on T lymphocytes.

The non-crosslinked form of trimeric recombinant 4-1 BBL can be used for inhibiting the proliferation of T-lymphocytes, while the crosslinked form can be used for activation and expansion of T lymphocytes.

SEQUENCE LISTING

<110>  INSERM (INSTITUT NATIONAL DE LA SANTE ET DE LA RECHERCHE
       MEDICALE)

<120>  RECOMBINANT TRIMERIC 4-1BBL.

<130>  MJP/ahF598/88

<160>  8

<170>  PatentIn version 3.3

<210>  1
<211>  206
<212>  PRT
<213>  Homo sapiens

<400>  1

Leu Ala Cys Pro Trp Ala Val Ser Gly Ala Arg Ala Ser Pro Gly Ser
1               5                   10                  15

Ala Ala Ser Pro Arg Leu Arg Glu Gly Pro Glu Leu Ser Pro Asp Asp
            20                  25                  30

Pro Ala Gly Leu Leu Asp Leu Arg Gln Gly Met Phe Ala Gln Leu Val
        35                  40                  45

Ala Gln Asn Val Leu Leu Ile Asp Gly Pro Leu Ser Trp Tyr Ser Asp
    50                  55                  60

Pro Gly Leu Ala Gly Val Ser Leu Thr Gly Gly Leu Ser Tyr Lys Glu
65                  70                  75                  80

Asp Thr Lys Glu Leu Val Val Ala Lys Ala Gly Val Tyr Tyr Val Phe
                85                  90                  95

Phe Gln Leu Glu Leu Arg Arg Val Val Ala Gly Glu Gly Ser Gly Ser
            100                 105                 110

Val Ser Leu Ala Leu His Leu Gln Pro Leu Arg Ser Ala Ala Gly Ala
        115                 120                 125

Ala Ala Leu Ala Leu Thr Val Asp Leu Pro Pro Ala Ser Ser Glu Ala
    130                 135                 140

Arg Asn Ser Ala Phe Gly Phe Gln Gly Arg Leu Leu His Leu Ser Ala
145                 150                 155                 160

Gly Gln Arg Leu Gly Val His Leu His Thr Glu Ala Arg Ala Arg His
                165                 170                 175

Ala Trp Gln Leu Thr Gln Gly Ala Thr Val Leu Gly Leu Phe Arg Val
            180                 185                 190

Thr Pro Glu Ile Pro Ala Gly Leu Pro Ser Pro Arg Ser Glu
        195                 200                 205

<210>  2
<211>  15
<212>  PRT
<213>  Artificial

<220>
<223>  Biotin acceptor peptide

<400>  2

Gly Leu Asn Asp Ile Phe Glu Ala Gln Lys Ile Glu Trp His Glu
1               5                   10                  15

<210>  3
<211>  26
<212>  DNA
<213>  Artificial

<220>
<223>  PCR primer

<400>  3
cgggatccct cgcctgcccc tgggcc
26

<210>  4
<211>  26
<212>  DNA
<213>  Artificial

<220>
<223>  PCR primer

<400>  4
gctctagatt attccgacct cggtga
26

<210>  5
<211>  48
<212>  PRT
<213>  Artificial

<220>

<223>  Biotin acceptor peptide with stating sequence

<400>  5

Thr Cys Cys Gly Gly Cys Cys Thr Gly Ala Ala Cys Gly Ala Cys Ala
1               5                   10                  15

Thr Cys Thr Thr Cys Gly Ala Gly Gly Cys Thr Cys Ala Gly Ala Ala
            20                  25                  30

Ala Ala Thr Cys Gly Ala Ala Thr Gly Gly Cys Ala Cys Gly Ala Ala
        35                  40                  45

<210>  6
<211>  17
<212>  PRT
<213>  Artificial

<220>
<223>  Biotin acceptor peptide with starting sequence

<400>  6

Met Ser Gly Leu Asn Asp Ile Phe Glu Ala Gln Lys Ile Glu Trp His
1               5                   10                  15

Glu

<210>  7
<211>  25
<212>  DNA
<213>  Artificial

<220>
<223>  PCR primer

<400>  7
gatccctcgc ctccccctgg gccgt
25

<210>  8
<211>  25
<212>  DNA
<213>  Artificial

<220>
<223>  PCR primer

<400>  8
acggcccagg gggaggcgag ggatc
25

## Description

The present invention relates to the production of soluble and crosslinked forms of the extracellular domain of 4-1BBL, and to their uses.

4-1BBL (CD137L), is a type II glycoprotein belonging to the tumor necrosis factor (TNF) superfamily. Interaction between 4-1BBL and its receptor 4-1BB provides a co-stimulatory signal for T lymphocyte proliferation and survival.

4-1BBL was first characterized in the mouse (I) and then in human (2); it is also disclosed in PCT WO 94/26290. The sequence of human 4-1BBL is available in databases (for instance it can be found in the Swiss-Prot/TrEMBL database under accession number P41273). It is 254 amino-acids-long, and comprises a cytoplasmic domain of 25 amino acids at the N-terminus of the polypeptide, followed by a transmembrane region of 23 amino-acids and an extracellular domain of 206 amino-acids (2).

The extracellular domain is also referred as "soluble 4-1BBL". It comprises a region highly conserved between the members of TNF superfamily, which is known as "TNF homology domain" (THD) which comprises the aminoacids responsible for the receptor binding. THDs share a similar tertiary structure and THDs of different members of TNF superfamily have been reported to associate to form trimeric proteins. In human 4-1BBL the THD is 162 amino-acids-long and is preceded by a 43 amino-acids-long tail.

4-1BBL expression is induced upon stimulation on various antigen-presenting cells such as B cells, monocytes and splenic dendritic cells as well as on T lymphocytes (for review, (3)). It interacts with 4-1BB (CD137), expressed almost exclusively on T cells following TCR stimulation (4) leading to T cell proliferation and cytokine production. 4-1BB ligation also promotes T cell survival and inhibits apoptosis via induction of the anti-apoptotic Bc1-X_{L} (5,6). While CD28/B7 interactions play a key role in the early phases of antigen-recognition, 4-1BBL stimulation seems implicated later in the primary response and during re-exposure to the antigen (7). Moreover, a report from Melero *et al.* (8) highlighted the importance of 4-1 BB/4-1 BBL interactions in antitumor immunity by showing that administration of agonistic anti 4-1BB to the mouse resulted in dramatic tumor regressions, even against low immunogenic tumors.

In spite of the growing interest in studying the roles of 4-1BB stimulation in T cell expansion, activation and survival, the use of recombinant 4-1BBL was only described in two reports (9,10) and in both, it is was mouse 4-1BBL that significantly differs from human 4-1BBL (only 36% homology at the protein level). Indeed, most of the *in vitro* and *in vivo* studies were performed either with a stimulatory anti-4-1BB mAb (8,11,12) or with cells transfected with 4-1BBL cDNA (13-15). In addition, although different recombinant human 4-1BBLs are commercially available today (Alexis, Chemicon, Ancell), they are poorly characterized biochemically and none of them are described as being able to co-stimulate T lymphocytes. Therefore, little evidence is available to date in the literature concerning the structure of 4-1BBL and its organisation to achieve T cell co-stimulation.

The inventors have thus undertaken to produce a soluble form of human 4-1BBL to determine its structure, its binding characteristics towards 4-1BB, to evaluate its functional activity, and to compare them with those of a commercially available recombinant human 4-1BBL.

They have constructed a soluble recombinant 4-1BBL comprising the human 4-1BBL extracellular domain coupled to a peptide (AviTag^{™}) having a site of enzymatic biotinylation. This protein was designed to include not only the THD of 4-1BBL but also the 43 aa-long tail. They have compared its properties with those of a commercially available 4-1BBL, FLAG-4-1BBL (Alexis) which is devoid of the 43 aa-long tail and wherein the FLAG tag peptide (8aa) is coupled to the THD through an undefined 11 aa linker. They found that while the AviTag-4-1BBL was able to refold as a trimer, it was not the case for FLAG-4-1BBL, which remained in a monomeric form.

They also observed that a cystein residue located at position 51 in the extracellular tail region could form a disulfide bond between two 4-1BBL monomers. Mutation of this cystein had no effect on trimerization, showing that said disulfide bond is not necessary for the formation of the trimer. It may however play a role in stabilizing 4-1BBL trimers.

They further observed that AviTag-4-1BBL trimers (with or without the intrachain disulfide bond) could bind recombinant 4-1BB with a much higher affinity than FLAG-4-1BBL but that surprisingly, this binding had no co-stimulating effect on CD3-stimulated T lymphocytes, and even inhibited the proliferation of CD3-stimulated PBMC. This inhibition of CD3-induced PBMC proliferation was also observed with soluble FLAG-4-1BBL.

In contrast, the inventors observed that AviTag-4-1BBL trimers (with or without the intrachain disulfide bond) immobilized onto streptavidine-coated beads, or crosslinked through a streptavidine bridge to form complexes consisting of two, three, or four trimeric units, became highly efficient in co-stimulating T lymphocyte proliferation. Although two crosslinked AviTag-4-1BBL trimeric units were sufficient to trigger co-stimulatory signals, complexes comprising three or four trimeric units led to enhanced co-stimulation. On the other hand, FLAG-4-1BBL immobilized onto anti-FLAG mAb-coupled beads remained non stimulatory.

Thus, the present invention provides means for regulating T-cells proliferation, which may be used in the therapy of several diseases.

More specifically, the present invention relates to a trimeric recombinant polypeptide, wherein each monomer of said trimeric polypeptide consists of a cross-linkable peptide domain followed by the whole extracellular domain of human 4-1BBL.

The "whole extracellular domain of human 4-1BBL" refers to a polypeptide having the following sequence (SEQ ID NO: 1): 
LACPWAVSGARASPGSAASPRLREGPELSPDDPAGLLDLRQGMFAQLVAQNVLLIDGPLSWYSDPGLAGVSLTG
GLSYKEDTKELVVAKAGVYYVFFQLELRRVVAGEGSGSVSLALHLQPLRSAAGAAALALTVDLPPASSEARNSA
FGFQGRLLHLSAGQRLGVHLHTEARARHAWQLTQGATVLGLFRVTPEIPAGLPSPRSE
 or to variants thereof having a mutation that does not hamper the formation of a 4-1BBL trimer. Examples of said variants are variants wherein the cystein residue at position 2 of SEQ ID NO: 1 is substituted by another aminoacid, thus preventing the formation of an intrachain disulfid bond between the monomers.

The cross-linkable peptide domain consists essentially of a "cross-linkable peptide", which is defined herein a peptide able to form a bridge with similar peptides, either directly, or preferably, through a connecting molecule. Examples of cross-linkable peptides are biotinylated peptides or peptides comprising an enzymatic biotinylation site, also known as "biotin acceptor peptides". Once biotinylated, said peptides can interact with streptavidin which plays the part of the connecting molecule, to form stable complexes comprising up to four peptides. Biotin acceptor peptides are disclosed for instance in U.S. Patents Nos. 5,723,584, 5,874,239 and 5,932,433. Once biotinylated, those peptides can be cross-linked through a streptavidin bridge. By way of example, the biotin acceptor peptide which was used for the construction of the cross-linkable domain of the trimeric recombinant 4-1BBL disclosed in the following examples is the 15-mer GLNDIFEAQKIEWHE, (SEQ ID NO: 2) preceded, for an efficient expression, by the starting sequence (MS), and followed by a 2 amino-acids linker (GS).

Other examples of cross-linkable peptides include StrepTag (16) or SBP (17) or NanoTag (18) which display a natural affinity for modified or native streptavidine and can thus be crosslinked by streptavidine or by ACRP30 which contains a collagen domain that associates naturally to form "bouquet-like" structures (19).

The invention also relates to a complex comprising at least two, preferably at least three trimeric recombinant polypeptides of the invention having their cross-linkable domains linked through a connecting molecule.

The invention also encompasses a solid support having trimeric recombinant polypeptides of the invention fixed thereto by their cross-linkable domains, through a connecting molecule coated on said solid support.

According to a preferred embodiment of said complex, or said solid support, said trimeric recombinant polypeptides have a biotinylated cross-linkable peptide domain, and said connecting molecule is streptavidine.

Solid supports that can be used in the present invention include for instance glass plates, plastic microtiter plates, microbeads, in particular glass or magnetic beads, colloidal matrices, and the like.

The invention also provides artificial antigen presenting cells comprising a solid support, for instance magnetic beads, having trimeric recombinant polypeptides of the invention fixed thereto as defined above, and also having attached thereto major histocompatibility complex (MHC) molecules, empty, or loaded with a chosen antigenic peptide.

Methods for attaching MHC molecules to solid supports are known in themselves; they are for instance disclosed in Bodinier et al, (20) Knabel et al, (16); Oelke et al,(21). Preferably, one will link the MHC molecule or a portion thereof comprising at least the extracellular domain to a cross-linkable peptide identical to the one used for constructing the recombinant polypeptides of the invention, thus facilitating the fixation of both the MHC molecules and the trimeric recombinant polypeptides of the invention to the same solid support.

Said artificial antigen presenting cells may optionally also comprise one or more other molecule(s) involved in T lymphocytes activation, attached in the same way to the same solid support. Such molecules may include for instance other members of the TNF ligand family such as OX40L, CD27L, CD30L or CD40L or other co-stimulatory molecules such as ICAM-1, ligand of LFA-1, LFA-3, ligand of CD2.

The invention also provides a method for *in vitro* activation and expansion of MHC restricted antigen-specific T lymphocytes wherein said method comprises contacting purified T lymphocytes or, advantageously, Peripheral Blood Mononuclear Cells (PBMC), with the cognate MHC molecule loaded with a chosen antigenic peptide, and, simultaneously, with a complex, or a solid support, bearing trimeric recombinant polypeptides of the invention, as indicated above. The antigenic peptide is a T-cell epitope of an antigen of interest, previously selected on the basis on its ability to be presented by the MHC molecule and to induce a T-cell response.

Said lymphocytes may be CD8+ lymphocytes: in this case, the MHC molecule is a MHC I molecule. They may also be CD4+ lymphocytes, and in this case the MHC molecule will be a MHC II molecule.

According to a preferred embodiment, said lymphocytes are contacted with artificial antigen presenting cells of the invention, loaded with the chosen antigenic peptide.

However, various alternatives are possible, provided that they allow the MHC molecule and the trimeric recombinant polypeptides of the invention to be contacted together with the lymphocytes: by way of non limitative examples, the MHC molecules may be on artificial antigen presenting cells, or coated on a solid support such as microbeads, or in the form of MHC tetramers; the trimeric recombinant polypeptides of the invention in fixed on microbeads and the trimeric recombinant polypeptides of the invention may be fixed on separate microbeads; or the MHC molecules may be in the form of MHC tetramers, and the trimeric recombinant polypeptides in the form of a complex of the invention, as defined above; or the MHC molecules may be in the form of MHC tetramers, and the trimeric recombinant polypeptides of the invention fixed on a solid support; or conversely, the trimeric recombinant polypeptides may be in the form of a complex of the invention, and the MHC molecules in the form of MHC tetramers.

According to a particularly preferred embodiment of a method of the invention, it comprises the culture of T lymphocytes, or of PBMCs, in presence of the complex or the solid support bearing trimeric recombinant polypeptides of the invention, and of the MHC molecule loaded with the chosen antigenic peptide. Preferably, said culture is performed in presence of artificial antigen presenting cells of the invention loaded with the chosen antigenic peptide.

Advantageously, said culture is performed during 7 to 30 days, preferably during about 14 days. Preferably, IL-15 is added to the culture medium during at least a part of the culture period. The inventors have observed that addition of IL-15 allowed an increase of the expression of 4-1 BB on the lymphocytes. IL-15 may be used at a final concentration in the culture medium of 5 to 40 ng/ml, preferably at about 10 ng/ml. Generally, it is added alone at the beginning of the culture, for 1 to 5 days, more preferably for about 3 days. At the end of this period, the addition of IL-15 is stopped, and IL-2 is added up to the end of the culture. IL-2 may be used at a final concentration in the culture medium of 10 to 150 UI/ml, preferably at about 50 UI/ml. Alternatively, IL7 may be used in replacement of IL-2.

The method of the invention allows to obtain a very important expansion of T lymphocytes, with a very high proportion of antigen-specific lymphocytes which have a memory-cell phenotype; further, they are very reactive and produce IL-2.

The invention also relates to a recombinant polynucleotide encoding a monomer of a trimeric polypeptide of the invention. Said nucleic acid molecule comprises a nucleotide sequence encoding a cross-linkable peptide domain, as defined above, linked to a nucleotide sequence encoding the whole extracellular domain of human 4-1BBL.

The invention also includes a vector comprising a polynucleotide of the invention. Preferably, said vector is an expression vector, wherein said polynucleotide is operatively linked to one or more control sequences allowing its expression in a prokaryotic or eukaryotic host cell. Said control sequences include at least a promoter allowing the transcription of the coding sequence of interest placed under its control; generally they also comprise a termination signal, ensuring termination of transcription and stabilization of the transcript. They can also include additional control elements such as enhancers. Optionally, said vector can also provide a sequence encoding a signal peptide allowing the secretion of the polypeptide of interest into the medium.

The invention also relates to a host cell transformed by a polynucleotide or a vector of the invention. Said host cell may be a prokaryotic cell, or a eukaryotic cell, including yeast, insect cells, plant cells, or mammalian cells.

The choice of the vector, of the control sequences, and of the additional elements that the vector may include depends mainly on the host-cell and of the expression system that one will choose to use. A number of expression systems, as well as methods for expressing recombinant polypeptides in large choice of host cells are well known to those skilled in the art.

The invention also provides a process for the production of a trimeric polypeptide of the invention, said process comprising culturing a host-cell containing an expression vector of the invention, under conditions allowing the expression of the monomers of the trimeric polypeptide of the invention, and recovering the trimeric polypeptide from the culture.

The invention also relates to medical uses of a trimeric polypeptides of the invention and more generally to medical uses of the extracellular domain of human 4-1BBL.

A shown above, cross-linked forms of a polypeptide of the invention, i.e. the above-defined complexes and solid supports, in particular coated beads, comprising polypeptides of the invention can be used for obtaining antigen-specific cytotoxic T-cells, useful in the treatment of tumoral diseases or viral diseases by adoptive immunotherapy.

They can thus be used for the treatment of almost any tumoral disease accessible to T lymphocyte infiltration such as for example, melanoma, colon cancer, breast cancer, and are more specifically interesting for those involving tumoral antigens against which specific cytotoxic T-cells are reputed to be difficult to obtain with classical methods. In the case of viral diseases, they can be used for instance for the treatment of HIV infection, HCV and CMV infections, of lymphoproliferative disease caused by Epstein-Barr virus.

On the other hand, non-cross-linked forms of a polypeptide of the invention, as well as trimers of the whole extracellular domain of human 4-1BBL (without the cross-linkable peptide), may be used for the treatment of diseases wherein it is desired to inhibit the proliferation of T-lymphocytes. Said diseases include for instance, graft rejection following transplantation, or autoimmune diseases such as type I diabetes, Hashimoto thyroiditis, multiple sclerosis, Crohn disease, and the like.

The present invention will be further illustrated by the following figures and by the detailed description which follows, which refers to non-limitative examples of obtention and use of trimeric recombinant polypeptides of the invention and compositions of the invention.

### FIGURE LEGENDS

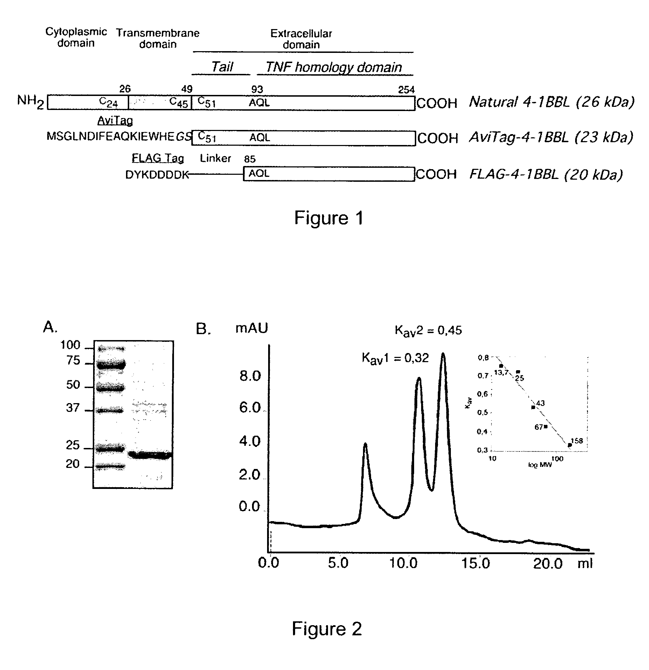

Fig. 1. Schematic representation of the different human 4-1BBL proteins studied. The AQL sequence corresponds to the beginning of the TNF homology domain (THD). In the AviTag-4-1BBL protein, the entire extracellular domain of human 4-1BBL (aa 49-254) was coupled to a biotin tag (AviTag) whereas in FLAG-4-1BBL, FLAG peptide (8aa) was coupled to the THD through an undefined 11aa linker.

Fig. 2. *A,* SDS-Page and Coomassie staining of 30 µg of inclusion bodies of WT AviTag-4-1BBL. AviTag-4-1BBL appeared as a major band around 23 kDa, the expected molecular weight of the monomer. *B,* Gel Filtration analysis of 10 µg of refolded AviTag-4-1BBL on a S200 column. The first peak corresponded to excluded aggregates. Kₐᵥ of the peaks are indicated and the calibration curve is shown in insert. Kₐᵥ2 corresponded to a molecular weight around 70 kDa, compatible with trimeric 4-1BBL while Kₐᵥ1, estimated around 140 kDa, may represent dimers of trimers.

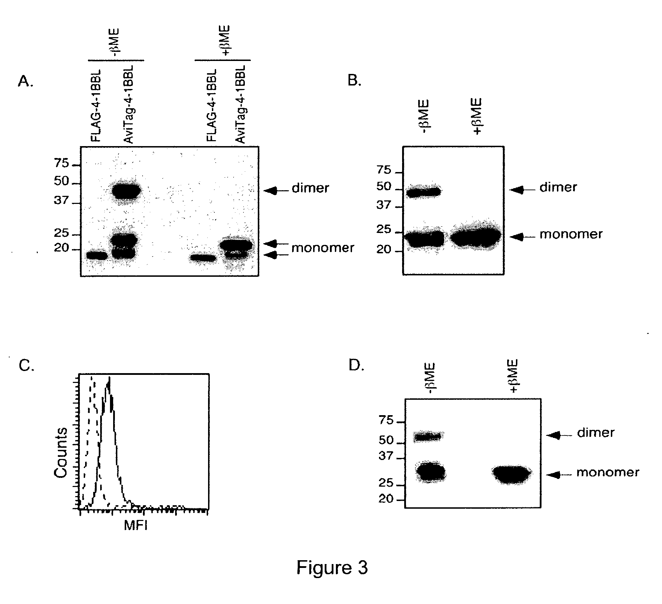

Fig. 3. *A,* Western blot analysis of purified WT AviTag-4-1BBL produced in E. Coli and FLAG-4-1BBL produced in HEK293 with a polyclonal goat anti-4-1BBL Ab. FLAG-4-1BBL appeared as a single band at the expected 20 kDa molecular weight. WT AviTag-4-1BBL dissociated into a monomer of 23 kDa and a dimer that disappeared under reducing conditions. The additional lower band represented a truncated form. *B,* Same analysis as in *A,* with WT AviTag 4-1BBL produced in S2 drosophila cells. Dimers containing a disulfide bond and monomers could be evidenced. *C,* Analysis of 4-1BBL expression on the T2 hybridoma cell line by flow cytometry after staining with a mouse anti 4-1BBL mAb. *D,* Same as in *A* with membrane-bound 4-1BBL from T2 cells. Triton X-114 extracts from 6 x 10⁷ T2 cells were precipitated and loaded in each well. Western blot revealed the presence of a dimer containing a disulfide bond.

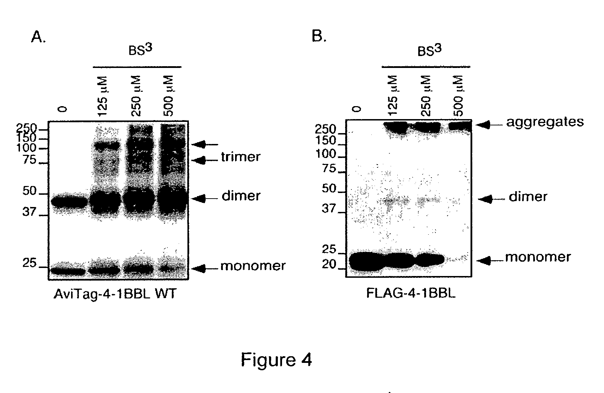

Fig. 4. *A,* Crosslinking of WT AviTag-4-1BBL with BS3 followed by western blot with goat anti 4-1BBL polyclonal Ab. Addition of increasing concentrations of BS3 resulted in the appearance of higher MW bands compatible with trimers and possibly dimers of trimers. *B,* Same as in *A* with FLAG-4-1BBL. Crosslinking generated weak dimeric forms and aggregates but no visible trimer.

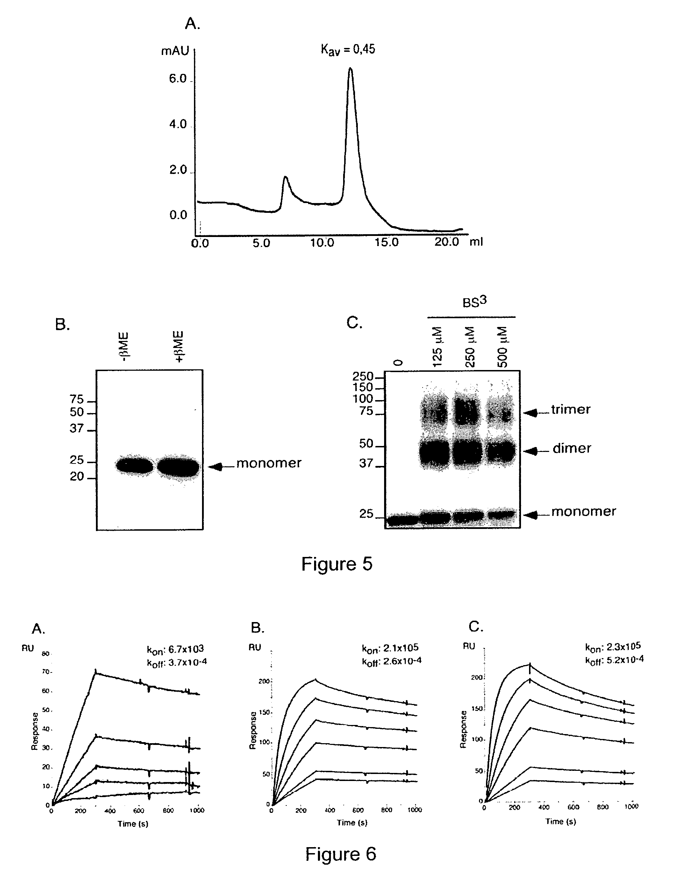

Fig. 5. *A,* Gel filtration analysis of C51S AviTag-4-1BBL after refolding. The major peak is compatible with a homotrimer. *B,* Western blot analysis of C51S AviTag-4-1BBL with goat anti 4-1BBL Ab. Mutation of the cystein abrogated dimer detection. *C,* Crosslinking of C51S AviTag-4-1BBL with BS³ and western blot with goat anti 4-1BBL Ab. Addition of increasing concentrations of BS³ resulted in the appearance of bands corresponding to dimers and trimers.

Fig. 6. Dynamic analysis of binding of AviTag-4-1BBLs (WT and C51S) and FLAG-4-1BBL to 4-1BB by BIAcore. Human 4-1BB/Fc was covalently coupled to the BIACore chip and a range of concentrations (from 0.725 to 72.46 nM) of the different ligands were assayed for binding at 25°C. Shown are sensograms of FLAG-4-1BBL (*A*), WT AviTag-4-1BBL (*B*) and C51S AviTag-4-1BBL (*C*). Calculated kinetic constants are indicated in each plot and correspond to the average value from two independent experiments.

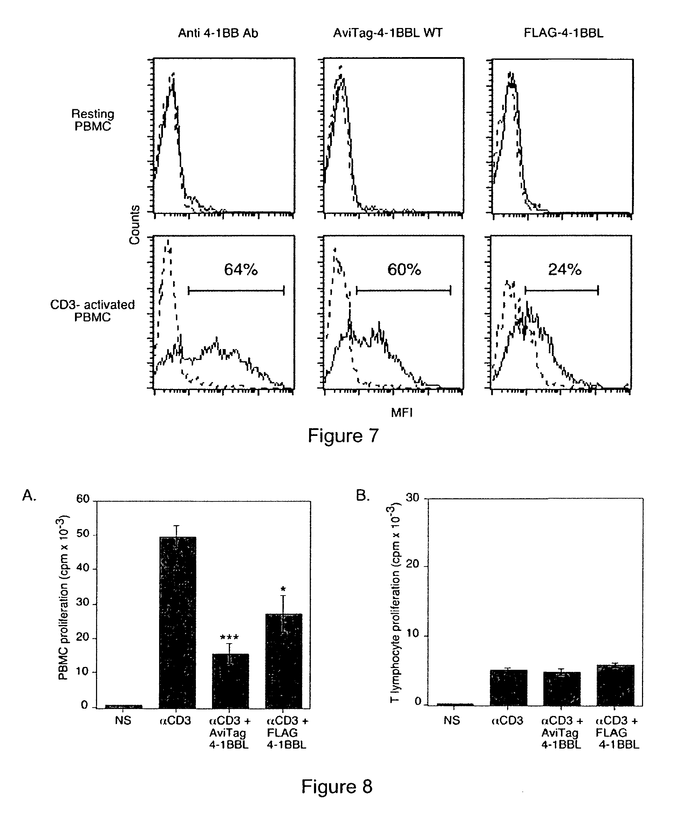

Fig. 7. Staining of 4-1BB on resting (*top panels*) or stimulated PBMC *(bottom panels)* using a control monoclonal anti 4-1BB Ab (*left panels*), AviTag-4-1BBL (10µg/ml) revealed by PE-conjugated streptavidine (*middle panels*) or FLAG-4-1BBL (10µg/ml) revealed by biotinylated anti FLAG mAb and PE-conjugated streptavidine *(right panels).* Dotted lines represent control staining with mouse IgG and secondary reagents. Percentage of positive cells are indicated.

Fig. 8. Effect of soluble WT AviTag-4-1BBL (10 µg/ml) and FLAG-4-1BBL (10 µg/ml) on CD3 stimulation. *A,* PBMC proliferation (105/well) induced by immobilized anti-CD3 mAb is inhibited by soluble WT AviTag-4-1BBL and by soluble FLAG-4-1BBL. *B,* Addition of soluble WT AviTag-4-1BBL or soluble FLAG-4-1BBL had no effect on the proliferation of purified T lymphocytes (10⁵/well) stimulated with anti-CD3 coated beads. In both panels, results are expressed as mean ± SEM of three independent experiments. ***p<0.001, *p<0.05 compared to CD3 alone

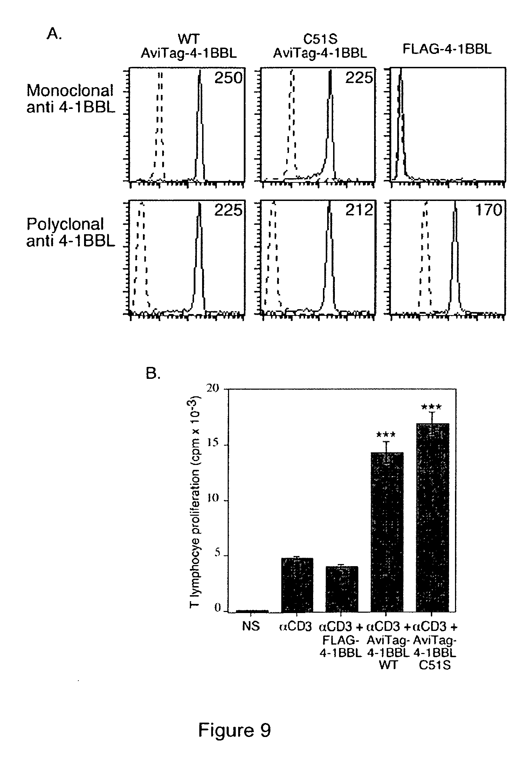

Fig. 9. *A,* Staining of streptavidine beads coated with WT (*left panels*), C51S (*middle panels*) or anti-FLAG beads with FLAG-4-1BBL (*right panels*) with a monoclonal mouse anti-4-1BBL Ab (*top panels*) or a polyclonal goat anti-4-1BBL Ab *(bottom panels).* Dotted lines represent control staining of uncoated beads. Mean fluorescence of stained beads is indicated in each panel. Monoclonal anti-4-1BBL did not recognize FLAG-4-1BBL. The high background staining of anti-FLAG beads alone was due to partial binding of the secondary anti-goat antibody to mouse anti-FLAG mAb. *B,* Effect of immobilized AviTag-4-1BBLs and FLAG-4-1BBL on T cell proliferation. Beads (1.3 x 10⁵/well) coated with WT (n=10 independent experiments) or C51S (n=3) AviTag-4-1BBL enhanced T cell proliferation but not FLAG-4-1BBL coated beads (n=3). Results are expressed as mean ± SEM. ***p<0.001 compared to CD3 alone.

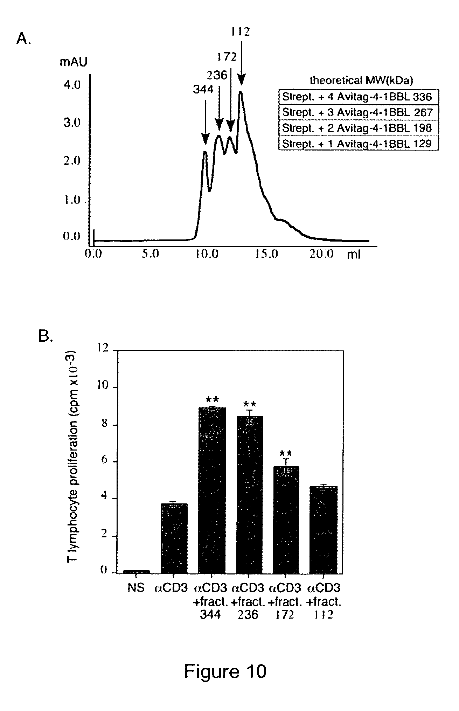

Fig. 10. A, S00 gel filtration analysis of oligomers of WT AviTag-4-1BBL. Trimeric AviTag-4-1BBL was mixed with a molar excess of streptavidine to obtain all degrees of multimerisation. Estimated molecular weight based on elution volume are indicated over each peak. The theorical molecular weight of the different oligomers are mentioned in the inset. *B,* Multimeric complexes (tetrameric, trimeric and dimeric) efficiently costimulated purified T lymphocyte proliferation. A narrow fraction of each peak represented in A was added to purified T lymphocytes activated for 6h with biotinylated anti-CD3 mAb coated beads. Results are expressed as mean ± SEM of three experiments. **p<0.01 compared to CD3 alone.

### MATERIALS AND METHODS

### Production of AviTag-4-1BBL as inclusion bodies in bacteria

The cDNA encoding the whole extracellular domain of 4-1BBL was obtained by reverse transcriptase (RT)-PCR amplification of T2 hybridoma RNA prepared using Trizol® (Gibco). Single-stranded cDNA was synthesized from 2 µg of total RNA using poly dT primer and M-MLV RT (200 UI, Gibco). The extracytoplasmic domain of 4-1BBL was amplified by PCR using the following primers: 5'-CGG GAT CCC TCG CCT GCC CCT GGG CC-3' and 5'-GCT CTA GAT TAT TCC GAC CTC GGT GA-3' (MWG-Biotech, France). The AviTag sequence (Avidity) TCC GGC CTG AAC GAC ATC TTC GAG GCT CAG AAA ATC GAA TGG CAC GAA (MSGLNDIFEAQKIEWHE) was ligated to the 5' end of 4-1BBL cDNA and the whole construct was inserted in the production vector pET24a (Invitrogen). AviTag-4-1BBL was produced as inclusion bodies in E. coli BL21(DE3) strain. The AviTag-4-1BBL protein (10 mg) was allowed to refold in 500 ml of folding buffer (0.4 M arginine, 100 mM Tris pH8, 2 mM EDTA pH8, 5 mM GSH, 0.5 mM GSSG, 0.005 % Tween 20 and a protease inhibitor cocktail ("Complete" Roche Diagnostics) for 72 h at 4°C under slow agitation, before concentration on a 10 kD cutoff membrane (Millipore). Biotinylation was performed using biotin protein ligase (Avidity) according to the manufacturer's instructions. After removal of free biotin on HiPrep desalting, AviTag-4-1BBL was purified by gel filtration on Superdex 200 HR 16/60 (Amersham). Mutation of AviTag-4-1BBL C51S was performed with 5'-GAT CCC TCG CCT CCC CCT GGG CCG T-3' and 5'-ACG GCC CAG GGG GAG GCG AGG GAT C-3' primers, using QuickChange Site-Directed Mutagenesis Kit (Stratagene) according to manufacturer's instructions. Production was performed as described above.

### Production of AviTag-4-1BBL in the insect cell line S2.

To allow secretion of AviTag-4-1BBL protein by S2 drosophila cells, we added the cDNA coding for the IL6 signal peptide to the 5' end of the bacterial construct and subcloned the whole construct in the pRmHa-3 expression vector which carries a copper-inducible promoter (a kind gift from Dr Goldstein). S2 cells were co-transfected with the pRmHa-3 and pCoblast which carries blasticidine resistance using the CaCl₂ method and selected with blasticidine for 3 weeks (25 µg/ml, Invitrogen). The bulk culture of blasticidine-resistant cells was cloned by limiting dilution using non transfected irradiated S2 drosophila cells as feeder. Clones were assayed for AviTag-4-1BBL production by intracytoplasmic staining with an anti 4-1BBL monoclonal Ab (BD Biosciences). Clone 1 was grown to a concentration of 5.10⁶/ml in serum-free culture medium (Invitrogen) and protein production was induced by addition of 0.75 mM CuSO₄ for 5 days at 27°C. The supernatant was concentrated on a VivaFlow concentrator cell, separated on a DEAE column, biotinylated as described above and the protein was finally purified by anion exchange chromatography on a MonoQ HR 10/10 column (Amersham). FLAG-4-1BBL was purchased from Alexis.

### SDS-Page and Western blot analysis of 4-1BBL proteins

Recombinant 4-1BBL proteins and native membrane 4-1BBL were analyzed by SDS-PAGE (12 % acrylamide gels). Gels were stained with saturated Coomassie blue R250 (Sigma) when appropriate. For immunoblotting, proteins were transferred onto a PVDF membrane (Immobilon-P, Millipore). AviTag-4-1BBL was detected with 1 µg/ml of a goat anti 4-1BBL polyclonal Ab (Santa Cruz biotechnology) followed by a HRP-conjugated rabbit anti goat Ab (1:15000, Santa Cruz biotechnology). Revelation was performed by ECL (Amersham).

In some instances, proteins were crosslinked prior to Western blotting using the nonreversible, homobifunctional and water soluble crosslinking agent BS³ (Pierce). To 40 pmol of protein, BS³ (10 mM in water) was added to the desired final concentration (125-500 µM) and crosslinking was allowed to process of 1 h at room temperature. The reaction was quenched with 1 M Tris pH 7.5.

For analysis of native 4-1BBL, membrane proteins were extracted from T2 cells using Triton X-114 as previously described (22). Briefly, 60.10⁶ T2 cells were lysed with 1 ml of lysis buffer (10 mM Tris pH 7.4, 150 mM NaCl, I % Triton X-114 (Sigma), a protease inhibitor cocktail ("Complete", Boehringer) for 30 min on ice. The lysate was centrifuged at 900 g for 10 min to remove cell debris and nuclei and overlaid on an iced-cooled sucrose cushion (6 % w/v sucrose, 10 mM Tris pH 7.4, 150 mM NaCl, 0.06 % Triton X-114). Clouding of the solution occurred after a 3 min incubation at 30°C. The tube was then centrifuged for 3 min at 300 g at room temperature to recover the detergent phase as an oily droplet at the bottom of the tube. The detergent phase enriched in membrane proteins was precipitated by MEOH/CHCl3 4:1 (v/v) before loading on a 12 % SDS-Page and immunoblotting as described above.

### Surface Plasmon Resonance Analyses

Binding experiments of the different recombinant 4-1BBL proteins to a recombinant human 4-1BB-Fc chimera (R&D Systems) were performed with a BIAcore 2000 optical biosensor (BIAcore AB, Uppsala, Sweden).The human 4-1BB/Fc protein was covalently coupled to a carboxymethyl dextran flow cell (CM5 BIAcore) as recommended by the manufacturer. The level of immobilization was set at 500 resonance units. Binding of purified mutant and native 4-1BBL was assayed at 25°C with concentrations ranging from 0.725 to 72.46 nM at a flow rate of 40 µl/min in HBS-EP buffer (0.01 M HEPES pH 7.4, 0.15 M NaCl, 3 mM EDTA, 0.005 % surfactant P20). Association was monitored for 5 min before initiating the dissociation phase for another 10 min with HBS-EP buffer. Regeneration of the flow cells was achieved by a I min pulse with 10 mM glycine-HCl at pH 1.8. The resulting sensorgrams were analysed using the BIA Evaluation software 3.2 (BIAcore AB).

### Multimerisation of the different recombinant 4-1BBLs

Coating of AviTag-4-1BBL (WT and C51S) onto M280 streptavidine magnetic beads was achieved by incubating decreasing concentrations of biotinylated proteins (ranging from 10 µg/ml to 5 ng/ml) with 67.10³ M280 magnetic microbeads (Dynal Biotech) for 2 h at room temperature under continuous agitation. Coating of FLAG-4-1BBL was performed in the same conditions using M280 tosylactivated magnetic beads covalently linked to an anti-FLAG mAb (clone M2, SIGMA). Saturation of the beads was checked by flow cytometry using either a mouse monoclonal PE-conjugated anti-4-1BBL (clone C65-485, BD Biosciences) or a goat anti 4-1BBL polyclonal Ab followed by an adsorbed FITC-conjugated donkey anti goat Ab as secondary reagent (Serotec). Saturation, estimated by the plateau of fluorescence, was obtained with 0.5 pg/bead of AviTag-4-1BBL and 2 pg/bead of FLAG-4-1 BBL.

Tetramerisation with streptavidine is usually achieved by mixing streptavidine (Sigma) and AviTag-4-1BBL at a molecular ratio of 1:4 for 1 h at room temperature. To obtain a mix of the different multimers (tetramers, trimers, dimers and monomers) we incubated AviTag-4-1BBL with a molar excess of streptavidine and then separated the different forms by gel filtration.

### Cell isolation and functional assays

Human peripheral blood mononuclear cell (PBMC) from healthy donors were isolated by Ficoll-Hypaque gradient centrifugation and resuspended in RPMI medium (Sigma) supplemented with 1 % L-glutamine and 8 % human serum. Purified T cells were obtained from fresh blood by negative selection using a RosetteSep isolation kit (StemCell Technologies). Purity of the T lymphocyte preparation was checked by flow cytometry with a FITC-conjugated anti CD3 mAb (BD Biosciences) and was always above 95%. T lymphocytes (10⁵/well, flat bottom 96-well plates, Falcon) were stimulated for 72 h with biotinylated anti CD3 mAb (clone UCHTI, BD Biosciences) immobilized on M280 streptavidine magnetic beads. To stimulate PBMC (10⁵/well), we used OKT3 (Orthoclone) immobilized in flat bottom 96-well plates by overnight incubation at 4°C in PBS instead of anti-CD3 coated beads because macrophages had a tendency to engulf the beads. All 4-1BB ligands were added after 6 h of CD3 stimulation to allow induction of 4-1BB expression on T lymphocytes. Proliferation was estimated by measuring incorporation of tritiated thymidine in quadruplicate during the last 16 h of culture.

Binding of the different recombinant 4-1BBLs to CD3-activated or resting T lymphocytes was evaluated at 48 h. Staining was performed at room temperature for I h using either PE-conjugated anti 4-1BB mAb (clone 4B4-1, BD Biosciences), AviTag-4-1BBL at 10 µg/ml revealed by PE-conjugated streptavidine at 0.5 µg/ml or FLAG-4-1BBL at 10 µg/ml revealed with a combination of mouse anti-FLAG mAb (10 µg/ml) and a FITC-conjugated goat anti-mouse F(ab')2 Ab (10 µg/ml). Staining of 4-1BBL on the T2 hybridoma was performed using PE-conjugated anti 4-1BBL (clone C65-485, BD Biosciences) for 45 min at 4°C. Flow cytometry was performed on a FacScan (BD Biosciences) and analyzed with the CellQuest Pro software (BD).

### Statistical Analysis

Results are expressed as mean ± SEM. All results were compared using ANOVA analysis followed by Tukey-Kramer post-tests. Post-tests were only performed when the ANOVA test showed a significant difference (p<0.05) between groups.

### EXAMPLE 1 : THE EXTRACELLULAR DOMAIN OF 4-1BBL FORMS A TRIMER CONTAINING A DISULFIDE BOND

To produce recombinant soluble 4-1BBL, we engineered a cDNA construct coding for the whole extracellular domain of human 4-1BBL coupled to a biotin tag, the AviTag. This construct included the 43 aa-long tail running from the membrane to the THD and thus differed from a commercially available FLAG-tagged 4-1BBL (Alexis) (Fig. 1). It should be noted that this tail contains a cystein close to the membrane.

We first produced the AviTag-4-1BBL protein of estimated mass 23kDa as inclusion bodies in *E. Coli.* Purity of inclusion bodies was above 95%, as evaluated by SDS-PAGE and Coomassie staining (Fig. 2A). After refolding and concentration, gel filtration analysis revealed two major peaks, peak 1 and 2 with molecular weights around 140 kDa and 70 kDa, respectively (Fig. 2B). Peak 2 was compatible with a trimeric form of AviTag-4-1BBL. SDS-PAGE electrophoresis followed by Western blotting of peak 2 showed that the trimeric form dissociated into a monomer and a dimer. The homodimer contained a disulfide bond as demonstrated by its disappearance under reducing conditions (Fig. 3A). Analysis of peak 1 by Western blot under reducing and non-reducing conditions showed an identical profile (not shown), suggesting that it corresponded to a multimeric complex of AviTag-4-1BBL linked by disulfide bonds. This form was discarded as wrongly folded since it turned out to be totally inactive in the functional assays described later. As expected, FLAG-4-1BBL appeared only as a monomer on SDS-PAGE since it lacks the cystein-containing region (Fig. 3A).

We wondered if the disulfide bond present in the trimeric form of AviTag-4-1BBL could have been artificially generated during the refolding of bacterial inclusion bodies. We thus produced soluble AviTag-4-1BBL in the Drosophila S2 expression system. In this system, the expressed protein is naturally processed inside the cell and then excreted in the culture medium. Analysis of AviTag-4-1BBL produced in S2 supernatants revealed a similar profile as that obtained with refolded inclusion bodies (Fig. 3B). This suggested that the disulfide bond was naturally formed in eucaryotic cells during AviTag-4-1BBL processing and export. To ascertain that the disulfide bond was indeed present in physiologically-expressed 4-1BBL, we performed Western blot analyses on Triton X-114 extracts from T2 hybridoma cells that constitutively express 4-1BBL (Fig. 3C) We obtained a similar profile, with a dimeric form that could be reduced by βME (Fig. 3D), thus confirming the presence of the disulfide bond in membrane 4-1BBL.

Finally, we performed crosslinking experiments with BS³ on refolded AviTag-4-1BBL and FLAG-4-1BBL to be able to visualize trimers on SDS-PAGE. Crosslinking of AviTag-4-1BBL with BS³ resulted in the appearance of bands of higher molecular weight among which one band around 69 kDa that would correspond to a trimer and a rather intense band around 140 kDa that may represent dimers of trimers (Fig. 4A). This observation further supported that AviTag-4-1BBL refolded as a trimer. In marked contrast, crosslinking of FLAG-4-1BBL revealed no trimeric forms but only a very weak signal compatible with dimers and big aggregates that did not penetrate into the gel (Fig. 4B). Considering that the dimeric forms probably originated from the dissociation by SDS of pre-existing and partially crosslinked trimers, our data suggest that only a very small fraction of FLAG-4-1BBL was in trimeric form (and thus undetectable as such after BS³ crosslinking) while most of it was monomeric.

### EXAMPLE 2 : THE DISULFIDE BOND STABILIZES TRIMERIC AVITAG-4-BBL

To investigate the role of the disulfide bond in 4-1BBL structure and function, we produced a mutated AviTag-4-1BBL where cystein was replaced by serine (C51S). Since the cystein residue is located well outside the THD of 4-1BBL, we anticipated that absence of the disulfide bond would not affect trimerization but may result in a less stable trimer. Indeed, after refolding, C51S AviTag-4-1BBL produced a peak compatible with a trimer but not the higher molecular weight form previously seen with the wild type (WT) AviTag-4-1BBL (Fig. 5A). Western blot analysis after SDS-PAGE showed that under non reducing conditions, the trimer dissociated into monomer only with no dimer, as expected (Fig. 5B) whereas crosslinking with BS³ revealed dimeric and trimeric forms of C51S AviTag-4-1BBL (Fig. 5C). We concluded that C51 S AviTag-4-1BBL could still refold as a trimer.

We next determined by surface plasmon resonance the kinetic constants of binding of native or mutated AviTag-4-1BBL and FLAG-4-1BBL to immobilized 4-IBB-Fc. The sensorgrams obtained with a range of concentrations (0.1 µg/ml to 5 µg/ml) of each of the three proteins are shown on figure 6. The first striking feature was that FLAG-4-1BBL displayed a much lower binding affinity to 4-1BB than native or mutated AviTag-4-1BBL (Kd of 55.2 nM for FLAG-4-1BBL *vs* 1.2 nM for WT and 2.3 nM for C51S AviTag-4-1BBL). This lower affinity mainly resulted from a 30-fold lower association rate (kₒₙ) for FLAG-4-1BBL as compared to WT AviTag-4-1BBL (6.7 x 10³ M⁻¹s⁻¹ *vs* 2.1 x 10⁵ M⁻¹s⁻¹, respectively) while the dissociation constants (k_{off}) were comparable (2.6 x 10⁻⁴ s⁻¹ for WT AviTag-4-1BBL vs 3.7 x 10⁻⁴ s⁻¹ for FLAG-4-IBBL). Since the association rate depends on the concentration of ligand while the dissociation constant does not, our data suggested that only a very small fraction of FLAG-4-1BBL had the proper conformation to interact with 4-1BB while most of it could not.

A comparison between WT and C51S AviTag-4-1BBL revealed that the two proteins had very similar association rates (2.1 x 10⁵ M⁻¹s⁻¹ *vs* 2.3 x 10⁵ M⁻¹s⁻¹, respectively) which is consistent with our hypothesis that the disulfide bond has no influence on the trimeric conformation. In marked contrast, they significantly differed in terms of dissociation, with C51S AviTag-4-1BBL dissociating twice as fast as WT AviTag-4-1BBL (5.2 x10⁻⁴ s⁻¹ vs 2.6 x10⁻⁴ s⁻¹, respectively) (Fig. 6). This suggested that the presence of the disulfide bond stabilized trimeric WT AviTag-4-1BBL.

### EXAMPLE 3: TRIMERIC 4-1BBL CO-STIMULATES T LYMPHOCYTES ONLY WHEN CROSSLINKED.

To investigate the functional properties of AviTag and FLAG-4-1BBL, we firstly checked their ability to recognize 4-1BB on CD3-stimulated T lymphocytes. Flow cytometry analyses revealed that biotinylated AviTag-4-1BBL stained a similar percentage of 4-1BB positive activated T cells (60%) than did the anti-4-1BB mAb used as positive control (64%), although with a lower MFI (Fig. 7). In contrast, staining with FLAG-4-1 BBL was too low to identify all positive cells.

We then tested the ability of the two proteins in soluble form to co-stimulate CD3-induced proliferation using either PBMC or purified T cells. The major difference between these two experimental designs is that immobilized anti-CD3 mAb alone is sufficient to elicit strong proliferation of PBMC (Fig. 8A) due to the presence of other cell types, such as macrophages and B cells, that can provide co-stimulation signals to T cells, but stimulates much less purified T lymphocytes proliferation (Fig. 8B). Soluble AviTag-4-1BBL dose-dependently inhibited the proliferation of CD3-stimulated PBMC with a maximal inhibition around 70% at 10 µg/ml but had no effect on CD3-stimulated T lymphocytes. Put together, these data strongly suggested that binding of soluble AviTag-4-1BBL to 4-1BB on T lymphocytes did not activate the costimulation pathways but instead efficiently prevented 4-1BB/4-1BBL interactions between T lymphocytes and APCs. Similarly, soluble FLAG-4-1BBL at 10 µg/ml inhibited CD3-induced PBMC proliferation which confirmed its ability to bind 4-1BB.

Since AviTag-4-1BBL showed no co-stimulatory activity in soluble form, we were prompted to test its effect on T cell proliferation following immobilization on a matrix. To this aim, we coated streptavidine beads with biotinylated AviTag-4-1BBL (either WT or C51S) or anti-FLAG mAb-coupled beads with FLAG-4-1BBL and controled saturation of the beads by immunofluorescence with an anti-4-1BBL mAb. Interestingly, we observed a similar intensity of staining of beads coated with WT or C51S AviTag-4-1BBL but no staining of beads coated with FLAG-4-1BBL (Fig. 9A). The use of a polyclonal anti-4-1BBL antibody allowed us to ascertain that FLAG-4-1BBL was indeed coated onto the beads (Fig. 9A). We then tested these beads coated with different forms of 4-1BBL for their ability to co-stimulate proliferation of purified T cells. As shown on figure 9B, addition of beads coated with WT or C51 S AviTag-4-1BBL resulted in a very significant increase in proliferation of CD3-stimulated T cells (4793 ± 426 cpm for CD3 alone *vs.* 14335 ± 1239 cpm for CD3+ WT AviTag-4-1BBL, n=10, p<0.001) while no stimulatory effect was observed with FLAG-4-1BBL-coated beads. Similar results were obtained with beads coated with WT AviTag-4-1BBL produced in S2 insect cells (data not shown). This demonstrated that, once crosslinked, AviTag-4-1BBL could stimulate T cell proliferation whereas FLAG-4-1BBL remained non stimulatory after crosslinking.

We finally performed crosslinking of WT AviTag-4-1BBL with a molar excess of streptavidine to obtain all multimeric forms, from tetramers to monomers (Fig. 10A). Although peaks were not very well separated by gel filtration, we tested narrow fractions corresponding to each peak on CD3-stimulated T lymphocytes. We observed that high complexes (tetramers and trimers) very efficiently co-stimulated proliferation, that dimers were slightly less active, while monomers were inactive (Fig. 10B). This confirmed the critical requirement of crosslinking to obtain a co-stimulatory effect of AviTag-4-1BBL on T cell proliferation.

### REFERENCES

1. Goodwin, R. G., Din, W. S., Davis-Smith, T., Anderson, D. M., Gimpel, S. D., Sato, T. A., Maliszewski, C. R., Brannan, C. I., Copeland, N. G., Jenkins, N. A., and et al. (1993) Eur J Immunol 23, 2631-2641
2. Alderson, M. R., Smith, C. A., Tough, T. W., Davis-Smith, T., Armitage, R. J., Falk, B., Roux, E., Baker, E., Sutherland, G. R., and Din, W. S. (1994) Eur J Immunol 24, 2219-2227
3. Vinay, D. S., and Kwon, B. S. (1998) Semin Immunol 10, 481-489
4. Kwon, B. S., and Weissman, S. M. (1989) Proc Natl Acad Sci U S A 86, 1963-1967
5. Lee, H. W., Park, S. J., Choi, B. K., Kim, H. H., Nam, K. O., and Kwon, B. S. (2002) J Immunol 169, 4882-4888
6. Starck, L., Scholz, C., Dorken, B., and Daniel, P. T. (2005) Eur J Immunol 35, 1257-1266
7. Bertram, E. M., Dawicki, W., Sedgmen, B., Bramson, J. L., Lynch, D. H., and Watts, T. H. (2004) J Immunol 172, 981-988
8. Melero, I., Shuford, W. W., Newby, S. A., Aruffo, A., Ledbetter, J. A., Hellstrom, K. E., Mittler, R. S., and Chen, L. (1997) Nat Med 3, 682-685
9. Saoulli, K., Lee, S. Y., Cannons, J. L., Yeh, W. C., Santana, A., Goldstein, M. D., Bangia, N., DeBenedette, M. A., Mak, T. W., Choi, Y., and Watts, T. H.(1998) J Exp Med 187, 1849-1862
10. Cannons, J. L., Lau, P., Ghumman, B., DeBenedette, M. A., Yagita, H., Okumura, K., and Watts, T. H. (2001) J Immunol 167, 1313-1324
11. Hurtado, J. C., Kim, Y. J., and Kwon, B. S. (1997) J Immunol 158, 2600-2609
12. Shuford, W. W., Klussman, K., Tritchler, D. D., Loo, D. T., Chalupny, J., Siadak, A. W., Brown, T. J., Emswiler, J., Raecho, H., Larsen, C. P., Pearson, T. C., Ledbetter, J. A., Aruffo, A., and Mittler, R. S. (1997) J Exp Med 186, 47-55
13. Gramaglia, I., Cooper, D., Miner, K. T., Kwon, B. S., and Croft, M. (2000) Eur J Immunol 30, 392-402
14. Wen, T., Bukczynski, J., and Watts, T. H. (2002) J Immunol 168, 4897-4906
15. Maus, M. V., Thomas, A. K., Leonard, D. G., Allman, D., Addya, K., Schlienger, K., Riley, J. L., and June, C. H. (2002) Nat Biotechnol 20, 143-148
16. Knabel M, Franz TJ, Schiemann M, Wulf A, Villmow B, Schmidt B, Bernhard H, Wagner H, Busch DH. (2002) Nat Med. 8, 631-637
17. Keefe AD, Wilson DS, Seelig B, Szostak JW. (2001) Protein Expr Purif. 23, 440-6
18. Lamla T, Erdmann VA. (2004) Protein Expr Purif., 33, 39-47
19. Schneider, P., Holler, N., Bodmer, J. L., Hahne, M., Frei, K., Fontana, A., and Tschopp, J. (1998) JExp Med, 187, 1205-1213
20. Bodinier M, Peyrat MA, Tournay C, Davodeau F, Romagne F, Bonneville M, Lang F. (2000) Nat Med., 6 , 707-10
21. Oelke M, Maus MV, Didiano D, June CH, Mackensen A, Schneck JP. (2003) Nat Med. May, 9, 619-24.
22. Bordier, C. (1981) JBiol Chem 256, 1604-1607

## Claims

1. A trimeric recombinant polypeptide, wherein each monomer of said trimeric polypeptide consists of a cross-linkable peptide domain followed by the whole extracellular domain of human 4-1BBL.

2. A trimeric recombinant polypeptide of claim 1, wherein the cross linkable-peptide domain comprises a biotin acceptor peptide.

3. A complex comprising at least two trimeric recombinant polypeptides of any of claims 1 or 2, having their cross-linkable domains linked through a connecting molecule.

4. A complex of claim 3, comprising three or four trimeric recombinant polypeptides of any of claims 1 or 2.

5. A solid support having trimeric recombinant polypeptides of any of claims 1 or 2 fixed thereto by their cross-linkable domains through a connecting molecule coated on said solid support.

6. A complex of any or claims 3. or 4, wherein said trimeric recombinant polypeptides have a biotinylated cross-linkable peptide domain, and said connecting molecule is streptavidine.

7. A solid support of claim 5, wherein said trimeric recombinant polypeptides have a biotinylated cross-linkable peptide domain, and said connecting molecule is streptavidine.

8. An artificial antigen presenting cell comprising a solid support of any of claims 5 or 7 further having attached thereto major histocompatibility complex (MHC) molecules, empty, or loaded with a chosen antigenic peptide.

9. A method for *in vitro* activation and expansion of MHC restricted antigen-specific T lymphocytes wherein said method comprises contacting simultaneously said lymphocytes with a cognate MHC molecule loaded with a chosen antigenic peptide, and with a complex of any of claims 3, 4 or 6, or a solid support of any of claims 5 or 7.

10. A method of claim 9, wherein said T lymphocytes are contacted with artificial antigen presenting cells of claim 8 loaded with the chosen antigenic peptide.

11. A method of any of claims 9 or 10, comprising the culture of T lymphocytes in presence of a complex of any of claims 3, 4 or 6, or of a solid support of any of claims 5 or 7 and of the MHC molecule loaded with the chosen antigenic peptide.

12. A method of claim 11, wherein said culture is performed during 7 to 30 days.

13. A method of any of claims 11 or 12, wherein IL-15 alone is added to the culture medium during at least a part of the culture period.

14. A method of claim 13, wherein IL-15 is used at a final concentration in the culture medium of 5 to 40 ng/ml

15. A method of any of claims 13 or 14, wherein IL-15 is added from the beginning of the culture, for1 to 5 days.

16. A method of claim 15 wherein IL-2 is added, following the addition of IL-15, and up to the end of the culture.

17. A method of claim 16, wherein IL-2 is be used at a final concentration in the culture medium of 10 to 150 UI/ml.

18. A recombinant polynucleotide encoding a monomer of a trimeric polypeptide of any of claims 1 or 2, wherein said polynucleotide comprises a nucleotide sequence encoding a cross-linkable peptide domain, as defined above, linked to a nucleotide sequence encoding the whole extracellular domain of human 4-1BBL.

19. A vector comprising a recombinant polynucleotide of claim 18.

20. A vector of claim 19 which is an expression vector.

21. A host cell transformed with an expression vector of claim 20.

22. A process for the production of a trimeric polypeptide of any of claims 1 or 2, said process comprising culturing a host-cell containing an expression vector of claim 1, and recovering said trimeric polypeptide from the culture.

23. The use of a complex of any of claims 3, 4 or 6, or of a solid support of any of claims 5 or 7 for obtaining antigen-specific cytotoxic T-cells.

24. The use of a trimeric recombinant polypeptide of any of claims I or 2, or of trimers of the whole extracellular domain of human 4-1BBL for preparing a therapeutic composition useful for the treatment of diseases wherein it is desired to inhibit the proliferation of T-lymphocytes, such as graft rejection following transplantation, or autoimmune diseases.
